Europäisches Patentamt

European Patent Office

Office européen des brevets

(19)

(11) Veröffentlichungsnummer: **0 031 106**
**B1**

(12)
# EUROPÄISCHE PATENTSCHRIFT

(45) Veröffentlichungstag der Patentschrift:
**01.08.84**

(51) Int. Cl.³: **A 61 B 6/02,** H 05 G 1/10

(21) Anmeldenummer: **80107873.4**

(22) Anmeldetag: **12.12.80**

(54) **Röntgenschichtgerät zur Herstellung von Transversalschichtbildern.**

(30) Priorität: **19.12.79 DE 2951222**

(43) Veröffentlichungstag der Anmeldung:
**01.07.81 Patentblatt 81/26**

(45) Bekanntmachung des Hinweises auf die Patenterteilung:
**01.08.84 Patentblatt 84/31**

(84) Benannte Vertragsstaaten:
**FR GB**

(56) Entgegenhaltungen:
**DE - A - 2 729 353**
**FR - A - 2 385 374**
**FR - A - 2 445 087**
**GB - A - 2 007 939**
**GB - A - 2 026 812**

(73) Patentinhaber: **SIEMENS AKTIENGESELLSCHAFT,**
**Berlin und München Wittelsbacherplatz 2,**
**D-8000 München 2 (DE)**

(72) Erfinder: *Franke, Kurt, Dr.-Ing., Lange Zeile 99,*
**D-8520 Erlangen (DE)**

## Beschreibung

Die Erfindung betrifft ein Röntgenschichtgerät zur Herstellung von Transversalschichtbildern eines Aufnahmeobjekts, mit einer Strahlenmessanordnung mit mindestens einer von einem Röntgengengenerator gespeisten Röntgenstrahlenquelle, die ein das Aufnahmeobjekt durchdringendes Röntgenstrahlenbündel erzeugt, dessen Querschnittsausdehnung senkrecht zur Schichtebene etwa gleich der Schichtstärke ist, mit einem Strahlenempfänger, der die Strahlenintensität hinter dem Objekt ermittelt, mit einem Drehrahmen zur Drehung der Röntgenstrahlenquelle in der Schichtebene für die Abtastung des Aufnahmeobjekts unter verschiedenen Projektionen und mit einem Messwertumformer für die Transformation der vom Strahlenempfänger gelieferten Signale in ein Schichtbild, bei dem der Hochspannungstransformator für die Röntgenröhre mit einer Frequenz betrieben wird, die höher als die Netzfrequenz ist (Mittelfrequenz) und mindestens die hochspannungsseitigen Bauelemente des Röntgengengenerators mit dem Mittelfrequenzerzeuger am Drehrahmen befestigt sind.

Ein Röntgenschichtgerät dieser Art ist in der DE-A-2 750 633 beschrieben. Bei diesem Röntgenschichtgerät ist eine schnelle Bewegung der Röntgenstrahlenquelle zur Abtastung eines Aufnahmeobjektes möglich, weil die hochspannungsseitigen Bauelemente des Röntgengengenerators mit dem Mittelfrequenzerzeuger am Drehrahmen befestigt sind und zusammen mit der Röntgenstrahlenquelle bei der Abtastung des Aufnahmeobjektes gedreht werden. Es brauchen also zu der Röntgenstrahlenquelle keine Hochspannungskabel geführt zu werden. Die Ausbildung des Röntgengengenerators als Mittelfrequenzgenerator führt dazu, dass der Hochspannungstransformator klein und leicht ausgebildet werden kann, so dass zur Abtastung eines Aufnahmeobjektes keine grossen Massen beschleunigt und abgebremst zu werden brauchen. Bei der Abtastung des Aufnahmeobjektes unter verschiedenen Projektionen ist es dabei sinnvoll, für jeweils eine Projektion die Röntgenröhre kurz einzuschalten, so dass sie beispielsweise bei 360 Projektionen pro Abtastvorgang 360mal kurzzeitig eingeschaltet wird.

Es ist auch bereits bekannt, bei einem Röntgenschichtgerät zur Herstellung von Transversalschichtbildern eines Aufnahmeobjektes eine Röntgenröhre vorzusehen, die während der Abtastung des Aufnahmeobjektes feststeht (DE-A-2 729 353). Die Röntgenröhre besteht dabei aus einem Glasring mit einer ringförmigen Anode und einer Vielzahl von Kathoden, die aufeinanderfolgend aktiviert werden. Zwar ist für jede Kathode je ein Steuergitter vorhanden, wodurch eine schnelle, schrittweise Weiterschaltung der Röntgenstrahlung möglich ist; hinsichtlich der Anordnung der erforderlichen Gittersteuervorrichtung ist dieser Schrift jedoch nichts entnehmbar. Der Heiztransformator für die Kathoden ist ortsfest angeordnet, und eine Speisung der Röntgenröhre mit Mittelfrequenz erfolgt nicht.

Der Erfindung liegt die Aufgabe zugrunde, ein Röntgenschichtgerät der eingangs genannten Art so auszubilden, dass eine sehr schnelle Ein- und Ausschaltung der Röntgenröhre bei Verwendung von Mittelfrequenz für die Speisung der Röntgenröhre erfolgen kann, so dass die Drehgeschwindigkeit des Drehrahmens hoch sein kann. Dabei soll der Hochspannungsgenerator nur dann eingeschaltet werden, wenn er auch Hochspannung an die Röntgenröhre liefern muss.

Diese Aufgabe ist erfindungsgemäss dadurch gelöst, dass als Strahlenquelle eine gittergesteuerte Röntgenröhre zusammen mit ihrem Heiztransformator in einem separaten Gehäuse vorgesehen ist, und dass eine Steuervorrichtung sowohl mit der gittergesteuerten Röntgenröhre als auch mit dem Hochspannungsgenerator in der Weise verbunden ist, dass die Aussteuerung des Steuergitters der Röntgenröhre gleichzeitig mit der Ein- oder Ausschaltung des Hochspannungsgenerators erfolgt. Mit der Zuführung von Steuerimpulsen zum Steuergitter der Röntgenröhre werden auch dem Hochspannungsgenerator auf dem Drehrahmen Steuerimpulse zugeführt, die die Hochspannung an der Röntgenröhre in dem Fall, in dem die Röntgenröhre keinen Strom führt, abschalten. Das Steuergitter an der Röntgenröhre hat den Vorteil, dass es auch eine sehr genaue Regelung des Röntgenröhrenstromes erlaubt. Über den Röntgenröhrenstrom ist dabei auch eine Regelung der Röntgenröhrenspannung aufgrund des Spannungsabfalles vor der Röntgenröhre möglich.

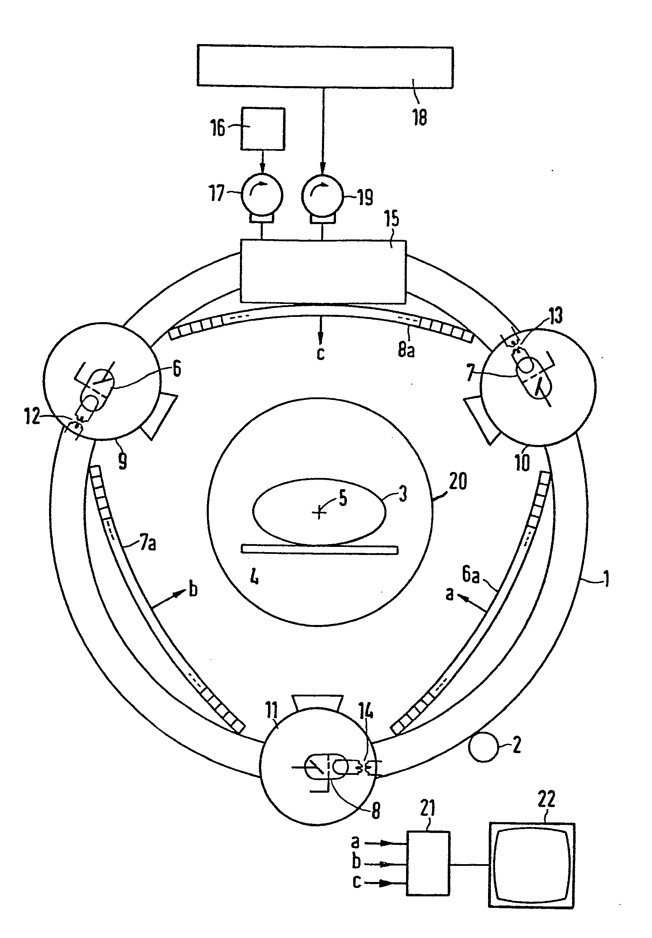

Die Erfindung ist nachfolgend anhand eines in der Zeichnung dargestellten Ausführungsbeispieles näher erläutert.

In der Zeichnung ist ein Drehrahmen 1 dargestellt, der durch eine Antriebsvorrichtung 2 zur Abtastung einer Transversalschicht eines Patienten 3, der auf einer Liege 4 liegt, um ein Zentrum 5 drehbar ist. Auf dem Drehrahmen 1 sind bei dem Beispiel drei Röntgenröhren 6 bis 8 angeordnet, von denen jede in einem Gehäuse 9 bis 11 vorgesehen ist. In den Gehäusen der gittergesteuerten Röntgenröhre 6 bis 8 sind auch die Heiztransformatoren 12 bis 14 angeordnet.

Zur Versorgung der Röntgenröhren 6 bis 8 mit Heiz- und Hochspannung ist auf dem Drehrahmen 1 ein Mittelfrequenz-Hochspannungsgenerator 15 befestigt, der von einem Netzgerät 16 seine Eingangsspannung über eine rotierende Stromkopplung 17 erhält. Ferner ist eine Gittersteuervorrichtung 18 vorhanden, die über eine rotierende Stromkupplung 19 den Hochspannungsgenerator 15 und die Röntgenröhren 6 bis 8 über deren Steuergitter ansteuert.

Jede der Röntgenröhren 6 bis 8 sendet ein fächerförmiges Röntgenstrahlenbündel aus, das mit seinen Randstrahlen einen Objektkreis 20 tangiert und auf einem gegenüberliegenden Strahlenempfänger 6a, 7a, 8a auftrifft. Jeder Strahlenempfänger 6a, 7a, 8a besteht aus einer Reihe von Einzeldetektoren, beispielsweise aus jeweils 512 Einzeldetektoren, von denen jeder die empfangene Strahlungsintensität in ein entsprechendes elektrisches Signal umwandelt. Jedes Röntgenstrahlenbündel ist in seiner Querschnittsausdehnung so bemessen, dass seine Stärke senkrecht zur Schichtebene gleich der Schichtstärke ist.

Die Ausgangssignale der Strahlenempfänger 6a, 7a, 8a, die bei jeder Projektion erzeugt werden, werden einem Computer 21 zugeführt, der daraus die Schwächungswerte vorbestimmter Punkte der untersuchten Transversalschicht bestimmt und ihre bildliche Wiedergabe auf einem Sichtgerät 22 bewirkt.

Die Untersuchung des Patienten 3 erfolgt in der Weise, dass der Drehrahmen 1 mit den darauf angeordneten Bauelementen um einen Winkel von 120° gedreht und bei jedem Grad nacheinander die Röntgenröhren 6, 7, 8 eingeschaltet werden. Auf diese Weise werden in jeder Winkelstellung des Drehrahmens 1 insgesamt 3 × 512 Detektor-Ausgangssignale und nach Abschluss einer vollständigen Abtastung 3 × 512 × 120 Detektorausgangssignale dem Computer 21 zugeführt. Die Schaltung der Röntgenröhren 6, 7, 8 erfolgt von der Gittersteuervorrichtung 18 aus über die Steuergitter. Dadurch ist auch bei einer schnellen Drehung des Drehrahmens 1 eine schnelle Schaltung möglich. In dem Fall, in dem eine Röntgenröhre keinen Strom führt, wird zweckmässigerweise über die Gittersteuervorrichtung 18 auch der Hochspannungsgenerator 15 so beeinflusst, dass die Hochspannung von der Röntgenröhre weggenommen wird.

Die Röntgenröhren 6, 7, 8 und ihre Heiztransformatoren 12, 13, 14 sind auf dem Drehrahmen 1 fest mit dem Hochspannungsgenerator 15, dessen Speisefrequenz im kHz-Bereich zwischen etwa 1 und 15 kHz liegt, verdrahtet.

Über die Steuergitter ist es auch möglich, den Röntgenröhrenstrom bei eingeschalteter Röntgenröhre zu regeln oder über den Röntgenröhrenstrom die Röntgenröhrenspannung konstant zu halten.

Die Anzahl der Röntgenröhren ist für die vorliegende Erfindung nicht wesentlich. Es ist also auch möglich, nur eine oder zwei Röntgenröhren am Drehrahmen 1 anzuordnen.

Bei dem Beispiel sind rotierende Stromkupplungen 17 und 19 zur Speisung und Steuerung des Hochspannungsgenerators 15 und der Röntgenröhren 6, 7, 8 vorgesehen. Es ist auch möglich, den Hochspannungsgenerator 15 über Kabel mit den zugeordneten Speise- und Steuereinrichtungen zu verbinden, die verhältnismässig leicht sein können, weil sie keine Hochspannungskabel sind. Auch der Anschluss der Strahlenempfänger 6a, 7a, 8a am Computer 21 kann über Niederspannungskabel erfolgen. Es ist aber auch denkbar, einen bekannten, stationären Detektorring als Strahlenempfänger vorzusehen, der die Röntgenröhren 6, 7, 8 aussen umschliesst oder in dem Drehrahmen 1 angeordnet ist und so verschwenkt wird, dass nur jeweils ein die zu empfangende Strahlung erfassender Teil in die Strahlung geschwenkt wird, wie dies in der DE-OS 2 714 759 beschrieben ist.

## Patentanspruch

Röntgenschichtgerät zur Herstellung von Transversalschichtbildern eines Aufnahmeobjektes, mit einer Strahlenmessanordnung mit mindestens einer von einem Röntgengenerator (15, 16) gespeisten Röntgenstrahlenquelle (6, 7, 8), die ein das Aufnahmeobjekt (3) durchdringendes Röntgenstrahlenbündel erzeugt, dessen Querschnittsausdehnung senkrecht zur Schichtebene etwa gleich der Schichtstärke ist, mit einem Strahlenempfänger (6a, 7a, 8a), der die Strahlenintensität hinter dem Objekt (3) ermittelt, mit einem Drehrahmen (1) zur Drehung der Röntgenstrahlenquelle (6, 7, 8) in der Schichtebene für die Abtastung des Aufnahmeobjektes (3) unter verschiedenen Projektionen und mit einem Messwertumformer (21) für die Transformation der vom Strahlenempfänger (6a, 7, 8a) gelieferten Signale in ein Schichtbild, bei dem der Hochspannungstransformator für die Röntgenröhre (6, 7, 8) mit einer Frequenz betrieben wird, die höher als die Netzfrequenz ist (Mittelfrequenz) und mindestens die hochspannungsseitigen Bauelemente des Röntgengenerators (15, 16) mit dem Mittelfrequenzerzeuger (15) am Drehrahmen (1) befestigt sind, dadurch gekennzeichnet, dass als Strahlenquelle eine gittergesteuerte Röntgenröhre (6, 7, 8) zusammen mit ihrem Heiztransformator (12, 13, 14) in einem separaten Gehäuse (9, 10, 11) vorgesehen ist und dass eine Steuervorrichtung (18) sowohl mit der gittergesteuerten Röntgenröhre (6, 7, 8) als auch mit dem Hochspannungsgenerator (15) in der Weise verbunden ist, dass die Aussteuerung des Steuergitters der Röntgenröhre (6, 7, 8) gleichzeitig mit der Ein- oder Ausschaltung des Hochspannungsgenerators (15) erfolgt.

## Claim

Tomographic X-ray apparatus for the production of transverse tomographs of an object to be recorded, having a radiation measuring arrangement with at least one X-ray source (6, 7, 8) fed by an X-ray generator (15, 16) and producing an X-ray beam which penetrates the object (3) to be recorded and whose cross-section expansion at right angles to the layer plane approximately equals the layer thickness, with a radiation receiver (6a, 7a, 8a) which determines the radiation intensity behind the object (3), with a rotating frame (1) which serves to rotate the X-ray source (6, 7, 8) in the layer plane for scanning the object (3) under different projections and having a measured value converter (21) for the transformation of the signals supplied from the radiation receiver (6a, 7a, 8a) into a tomograph, wherein the high-voltage transformer for the X-ray tube (6, 7, 8) is operated at a frequency higher than the mains frequency (medium frequency) and at least the high-voltage-end components of the X-ray generator (15, 16) together with the medium frequency generator (15) are secured to the rotating frame (1), characterised in that there is arranged as a radiation source in a separate housing (9, 10, 11) a grid-controlled X-ray tube (6, 7, 8) together with its heating transformer (12, 13, 14) and that a control device (18) is connected both to the grid-controlled X-ray tube (6, 7, 8) and also to the high-voltage generator (15) in such a manner that the control grid of the X-ray tube (6, 7, 8) is simultaneously controlled with the switching on or off of the high-voltage generator (15).

## Revendication

Appareil de tomographie pour la réalisation de tomographies transversales d'un objet de prise de vue, comportant un dispositif de mesure du rayonnement possédant au moins une source de rayons X (6, 7, 8) alimentée par un générateur radiologique (15, 16) et qui produit un faisceau de rayonnement traversant l'objet de prise de vues (3) et dont l'étendue en coupe transversale perpendiculairement au plan de la couche est égale approximativement à l'épaisseur de cette dernière, et possédant un récepteur de rayonnement (6a, 7a, 8a) qui détermine l'intensité du rayonnement derrière l'objet (3), un cadre tournant (1) servant à faire tourner la source de rayons X (6, 7, 8) dans le plan de la couche en vue de réaliser l'exploration de l'objet de prise de vue (3) suivant différentes projections, et un transformateur de valeurs de mesure (21) servant à transformer les signaux délivrés par le récepteur de rayonnement (6a, 7a, 8a) en une tomographie, et dans lequel le transformateur haute tension pour le tube à rayons X (6, 7, 8) fonctionne à une fréquence qui est supérieure à la fréquence du réseau (moyenne fréquence) et dans lequel au moins les composants du générateur radiologique (15, 16) situés du côté de la haute tension sont fixés ainsi que le générateur à moyenne fréquence (15) sur le cadre tournant (1), caractérisé par le fait qu'il est prévu comme source de rayonnement un tube à rayons X (6, 7, 8) commandé par sa grille disposé ainsi que son transformateur de chauffage (12, 13, 14) dans un boîtier séparé (9, 10, 11), et qu'un dispositif de commande (16) est relié aussi bien aux tubes à rayons X (6, 7, 8) commandés par la grille qu'au générateur haute tension (4) de sorte que la modulation de la grille de commande du tube à rayons X (6, 7, 8) est réalisée en même temps que le branchement ou le débranchement du générateur à haute tension (15).